# EUROPEAN PATENT SPECIFICATION

(11) **EP 3 871 588 B1**
(45) Date of publication and mention of the grant of the patent: **25.06.2025**
(21) Application number: 21155485.2
(22) Date of filing: 05.02.2021
(51) Int. Cl.: A61B 5/00, A61B 5/021, A61B 5/022, A61B 5/024

(54) **ELECTRONIC DEVICE AND METHOD FOR ESTIMATING BIO-INFORMATION**
ELEKTRONISCHE VORRICHTUNG UND VERFAHREN ZUR SCHÄTZUNG VON BIO-INFORMATIONEN
DISPOSITIF ÉLECTRONIQUE ET PROCÉDÉ D'ESTIMATION D'INFORMATIONS BIOLOGIQUES

(30) Priority: 07.02.2020 KR 20200015262; 05.06.2020 KR 20200068451
(43) Date of publication of application: 01.09.2021
(73) Proprietor: Samsung Electronics Co., Ltd., Suwon-si, Gyeonggi-do 16677 (KR)
(72) Inventor: KO, Byung Hoon, Gyeonggi-do (KR); KIM, Youn Ho, Gyeonggi-do (KR); NOH, Seung Woo, Gyeonggi-do (KR); PARK, Jong Hoon, Seoul (KR); AHN, Sung Mo, Gyeonggi-do (KR); YEOM, Eun Ju, Gyeonggi-do (KR); LEE, Yun Ki, Gyeonggi-do (KR); LEE, Jong Wook, Gyeonggi-do (KR); LEE, Tak Hyung, Seoul (KR); HWANG, Jeong Eun, Gyeonggi-do (KR)
(74) Representative: Grünecker Patent- und Rechtsanwälte PartG mbB

(56) References cited:
- EP-A1- 3 476 280
- WO-A1-2012/099535
- WO-A1-2020/009496
- US-A1- 2019 008 399
- ANAND CHANDRASEKHAR ET AL: "Smartphone-based blood pressure monitoring via the oscillometric finger-pressing method", SCIENCE TRANSLATIONAL MEDICINE, vol. 10, no. 431, 7 March 2018 (2018-03-07), US, pages eaap8674, XP055463286, ISSN: 1946-6234, DOI: 10.1126/scitranslmed.aap8674

## Description

### BACKGROUND

### 1. Field

The following description relates to an electronic device and bio-information estimation technology of the electronic device.

### 2. Description of Related Art

Generally, methods of non-invasively measuring blood pressure without damaging a human body include a method to measure blood pressure by measuring a cuff-based pressure and a method to estimate blood pressure by measuring a pulse wave without the use of a cuff. A Korotkoff-sound method is one of cuff-based blood pressure measurement methods, in which a pressure in a cuff wound around an upper arm is increased and blood pressure is measured by listening to the sound generated in the blood vessel through a stethoscope while decreasing the pressure. Another cuff-based blood pressure measurement method is an oscillometric method using an automated machine, in which a cuff is wound around an upper arm, a pressure in the cuff is increased, a pressure in the cuff is continuously measured while the cuff pressure is gradually decreased, and blood pressure is measured based on a point where a change in a pressure signal is large. Cuffless blood pressure measurement methods generally include a method of measuring blood pressure by calculating a pulse transit time (PTT) and a method a pulse wave analysis (PWA) method of estimating blood pressure by analyzing a shape of a pulse wave.

US 2019/008399 A1 discloses a system and method for cuff-less blood pressure measurement in a mobile device. The blood pressure measurement is performed at the fingertip of a subject and reflectance-mode photoplethysmography can be used for this measurement.

EP 3 476 280 A1 discloses an apparatus for measuring bio-information including a pulse wave sensor configured to emit light having a plurality of wavelengths onto an object, and to detect a multi-wavelength pulse wave signal from the object, and a processor configured to obtain a contact pressure signal based on the multi-wavelength pulse wave signal, the contact pressure signal indicating a pressure between the object and the pulse wave sensor, and to generate information regarding a measurement state of the object based on the contact pressure signal.

WO 2012/099535 discloses an optical measurement device providing an illumination and detection assembly configured to generate and detect light of a predetermined wavelength range in the form of a photoplethysmography (PPG) signal, as well as a pressure detection assembly configured to detect an amount of pressure applied to the measurement device by the user being measured. A feedback unit, such as a portable display device, can be coupled to the measurement device to provide the user with real-time feedback of the detected PPG signal and level of applied pressure so that the user may adjust the amount of applied pressure to change the quality of the detected PPG signal.

The publication of Anand Chandrasekhar et al. "Smartphone-based blood pressure monitoring via the oscillometric finger-pressing method", Science Translational Medicine, vol. 10, no. 431, 7 March 2018 (2018-03-07), page eaap8674 discloses smartphone-based blood pressure monitoring via the oscillometric finger-pressing method.

WO 2020/009496 discloses a biometric information measurement device and method.

It is the object of the present invention to provide an improved method for estimating bio-information in a precise manner.

This object is solved by the subject matter of the independent claims.

Embodiments are defined by the dependent claims.

This summary is provided to introduce a selection of concepts in a simplified form that are further described below in the Detailed Description. This summary is not intended to identify key features or essential features of the claimed subject matter, nor is it intended to be used as an aid in determining the scope of the claimed subject matter.

Additional aspects will be set forth in part in the description which follows and, in part, will be apparent from the description, or may be learned by practice of the presented embodiments.

According to an aspect of an example embodiment, an electronic device may include a main body; a sensor part disposed on a side of the main body; and a processor configured to control a display to display a first graphical object related to a contact state of a finger based on information related to the contact state of the finger received from the sensor part before measurement of a bio-signal; and control the display to display a second graphical object related to a contact force of the finger based on information related to the contact force of the finger received from the sensor part during measurement of the bio-signal.

The sensor part may include an optical sensor comprising a light source configured to emit light to the finger in contact with a finger contact surface and a photodetector configured to detect light scattered or reflected from the finger, and a force sensor configured to measure the contact force based on the finger contacting the finger contact surface.

In response to receiving a request for estimating bio-information, the processor is further configured to control the display to display the first graphical object representing an appearance of the main body and the sensor part, and a third graphical object representing an appearance of the finger that is in normal contact with the sensor part in the first graphical object.

The processor is further configured to control the display to display the first graphical object and repeatedly display or eliminate the third graphical object at least one or more times after a predetermined period of time.

The processor is further configured to determine whether the contact state of the finger is normal based on the information related to the contact state received from the sensor part.

The processor is further configured to control the display to display a third graphical object to induce a user to exert a force with the finger toward the sensor part based on the contact state being normal.

The third graphical object represents an appearance of the finger that repeatedly moves from a predetermined position spaced apart from the sensor part of the main body to a position of the sensor part.

The third graphical object includes an arrow directed toward the sensor part.

The processor is further configured to control the display to display a fourth graphical object indicating that the contact state is normal based on the contact state being normal.

The processor is further configured to display at least one of a fifth graphical object indicating that the contact state is abnormal, a sixth graphical object visually displaying a reason for the contact state being abnormal, and text describing a reason for the contact state being abnormal based on the contact state being abnormal.

The processor is further configured to display a seventh graphical object representing a range of a reference contact force that the finger is to exert to the sensor part based on the contact state being normal.

In response to receiving the contact force from the sensor part, the processor is further configured to control the display to display an eighth graphical object representing the contact force.

The processor is further configured to control the display to display the seventh graphical object, and display a gamified screen in which the eighth graphical object moves along the seventh graphical object in response to the contact force being received in real-time from the sensor part.

The processor is further configured to control a speaker to output a warning sound or control the display to display a ninth graphical object to warn a user based on the contact force being outside of the range of the reference contact force.

The processor is further configured to extract a feature based on the bio-signal measured by the sensor part, and estimate bio-information based on at least one of the extracted feature and the contact force.

According to an aspect of an example embodiment, a method of estimating bio-information which is performed by an electronic device comprising a sensor part disposed on a side of a main body and a processor may include acquiring, by the sensor part, a contact state of a finger before measurement of a bio-signal; controlling a display, by the processor, to display a first graphical object related to the contact state of the finger; acquiring, by the sensor part, a contact force of the finger during the measurement of the bio-signal; and controlling the display, by the processor, to display a second graphical object related to the contact force of the finger.

The method may include receiving a request for estimating bio-information; and controlling the display to display the first graphical object representing an appearance of the main body including the sensor part and a third graphical object representing an appearance of the finger that is in normal contact with the sensor part in the first graphical object.

The controlling the display to display the first graphical object related to the contact state comprises determining whether the contact state of the finger is normal based on the contact state.

The controlling the display to display the first graphical object related to the contact state comprises controlling the display to display a third graphical object to induce a user to exert a force with the finger toward the sensor part based on the contact state being normal.

The controlling the display to display the first graphical object related to the contact state comprises controlling the display to display at least one of a fifth graphical object indicating that the contact state is abnormal, a sixth graphical object visually displaying a reason for the contact state being abnormal, and text describing a reason for the contact state being abnormal based on the contact state being abnormal.

The controlling the display to display the second graphical object related to the contact force comprises displaying a seventh graphical object representing a range of a reference contact force that the finger is to exert to the sensor part based on the contact state being normal.

The controlling the display to display the second graphical object related to the contact force comprises, in response to receiving the contact force from the sensor part, controlling the display to display an eighth graphical object representing the contact force.

The method may include acquiring a bio-signal by the sensor part; and estimating bio-information based on the bio-signal and the contact force.

An electronic device may include a main body; a sensor part disposed on a side of the main body; and a processor provided inside the main body, electrically connected to the sensor part, and configured to control the sensor part, and process data received from the sensor part, wherein the sensor part comprises a housing disposed to be partially externally exposed from the side of the main body, a finger contact surface formed on an exposed surface of the housing to allow a finger to contact the finger contact surface, and an optical sensor disposed inside the housing.

The finger contact surface includes a convexly curved shape along a direction parallel to a length direction of the finger that is placed on and in contact with the finger contact surface.

The finger contact surface includes a convexly curved shape along a direction perpendicular to a length direction of the finger, or includes a shape having a flat top and curved surfaces on both sides.

The finger contact surface comprises a first light transmissive region and second light transmissive region that are formed on sides of the finger contact surface, and a third light transmissive region formed between the first light transmissive region and the second light transmissive region.

The optical sensor comprises a first light source, a second light source, and a photodetector provided between the first light source and the second light source.

The housing comprises a first light path configured to direct light emitted from the first light source toward the finger through the first light transmissive region, a second light path configured to direct light emitted from the second light source toward the finger through the second light transmissive region, and a third light path configured to direct light scattered or reflected from the finger toward the photodetector through the third light transmissive region.

The housing further comprises partition walls formed between the first light path and the second light path, and between the second light path and the third light path.

The electronic device further comprises a force sensor provided in the housing, and configured to measure a contact force applied by the finger to the finger contact surface.

The processor is configured to control the sensor part to operate in a general mode or in a bio-information estimation mode; and switch a mode of the sensor part to the bio-information estimation mode based on a user manipulation of the sensor part or an input of a request for estimating bio-information through a display mounted in the main body based on the sensor part being in the general mode.

Other features and aspects will be apparent from the following detailed description, the drawings, and the claims.

### BRIEF DESCRIPTION OF THE DRAWINGS

The above and other aspects, features, and advantages of certain embodiments of the present disclosure will be more apparent from the following description taken in conjunction with the accompanying drawings, in which:
FIG. 1 is a diagram illustrating an electronic device according to an embodiment;
FIGS. 2A to 5 are diagrams for describing a structure of a sensor part according to an embodiment;
FIGS. 6 and 7 are block diagrams illustrating an electronic device according to embodiments;
FIGS. 8 to 10 are diagrams illustrating embodiments in which graphical objects related to a contact state are displayed;
FIGS. 11A to 12B are diagrams illustrating embodiments in which graphical objects related to a contact force are displayed;
FIGS. 12C and 12D are diagrams for describing an example of measuring blood pressure based on oscillometry; and
FIG. 13 is a flowchart illustrating a method of estimating bio-information according to an embodiment.

Throughout the drawings and the detailed description, unless otherwise described, the same drawing reference numerals will be understood to refer to the same elements, features, and structures. The relative size and depiction of these elements, features, and structures may be exaggerated for clarity, illustration, and convenience.

### DETAILED DESCRIPTION

Details of example embodiments are provided in the following detailed description with reference to the accompanying drawings. The disclosure may be understood more readily by reference to the following detailed description of example embodiments and the accompanying drawings. The disclosure may, however, be embodied in many different forms and should not be construed as being limited to the embodiments set forth herein. Rather, these embodiments are provided so that the disclosure will be thorough and complete and will fully convey the concept of the invention to those skilled in the art, and the invention will only be defined by the appended claims. Like reference numerals refer to like elements throughout the specification.

It will be understood that, although the terms "first," "second," etc. may be used herein to describe various elements, these elements should not be limited by these terms. These terms are only used to distinguish one element from another. Also, the singular forms of terms are intended to include the plural forms of the terms as well, unless the context clearly indicates otherwise. In the specification, unless explicitly described to the contrary, the word "comprise," and variations such as "comprises" or "comprising," will be understood to imply the inclusion of stated elements but not the exclusion of any other elements. Terms such as "unit" and "module" denote units that process at least one function or operation, and they may be implemented by using hardware, software, or a combination of hardware and software.

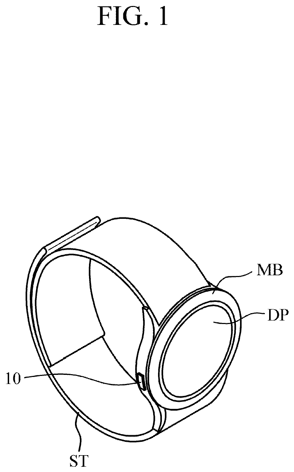

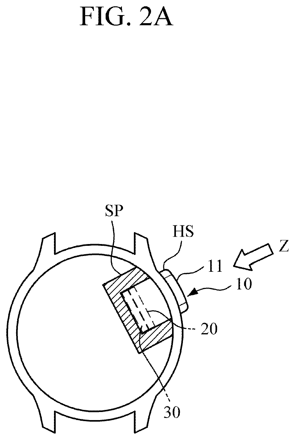

FIG. 1 is a block diagram illustrating an electronic device according to an embodiment. FIGS. 2A to 5 are diagrams for describing a structure of a sensor part according to an embodiment. Example embodiments of a structure of an electronic device 100 will be described with reference to FIGS. 1 to 5.

The electronic device 100 according to example embodiments may be a smart watch or a smart band-type wearable device. However, the electronic device 100 is not limited thereto, and may be a mobile device, such as a smartphone or a tablet personal computer (PC).

Referring to FIG. 1, the electronic device 100 may include a main body MB and a strap ST.

The main body MB may include modules for performing general functions of the electronic device 100 and a sensor part 10 for estimating bio-information. A battery may be embedded in the main body MB or the strap ST to supply power to various modules. The strap ST may be connected to the main body MB. The strap ST may be flexible so as to be bent around a user's wrist. The strap ST may include a first strap and a second strap that is separated from the first strap. Respective ends of the first strap and the second strap may be connected to each end of the main body MB, and the first strap and the second strap may be fastened to each other using fastening means formed on the other sides thereof. In this case, the fastening means may be formed as Velcro fastening, pin fastening, or the like, but is not limited thereto. In addition, the strap ST may be formed as one integrated piece, such as a band, which is not separated into pieces.

A display DP may be disposed on a top surface of the main body MB to visually display various types of information. The display DP may include a touch screen panel capable of receiving a touch input of a user.

The sensor part 10 is disposed on the side of the main body MB, possibly in the form of a button. The sensor part 10 may operate in a bio-information estimation mode and in a general mode under the control of a processor. When operating in the bio-information estimation mode, the sensor part 10 may acquire force information applied by an object to the sensor part 10 when the object is in contact with the sensor part 10. Also, when the object is in contact with the sensor part 10, the sensor part 10 may acquire light information reflected or scattered from the object. When operating in the general mode, the sensor part 10 may perform a user interface function for controlling general functions of the electronic device 100, for example, selection/execution of an application, adjustment of a graphical user interface (GUI) of the display DP, and the like.

Referring to FIG. 2A, the sensor part 10 may include a housing HS. In addition, the sensor part 10 may include an optical sensor 20 and a force sensor 30 which are disposed inside of the housing HS or at a lower end of the housing HS.

The housing HS may have a part in the form of a button, which is externally exposed through the side of the main body MB. For example, a supporter SP inside the main body MB may support the housing HS from at least one of the periphery and the lower end of the housing HS. In the embodiment of FIG. 2A, the supporter SP is illustrated as surrounding the housing HS inside the main body MB, but this is merely an example. An additional structure for preventing the housing HS from being dislodged from the main body MB may be further included in the housing HS or inside the main body MB.

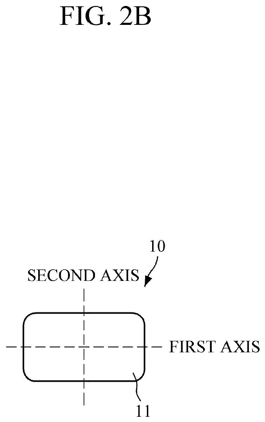

The housing HS may include a finger contact surface 11 which contacts a finger that is placed on and in contact with the finger contact surface 11. FIG. 2B is a diagram illustrating an embodiment showing the shape (hereinafter referred to as a "plan view") of the finger contact surface 11 when the sensor part 10 of FIG. 2A is viewed in a direction (Z direction) perpendicular to the finger contact surface 11. In the plan view of the finger contact surface 11, a first axis traversing the center of the finger contact surface 11 may be a long axis and a second axis traversing the center of the finger contact surface 11 in a different direction from that of the first axis may be a short axis. The first axis and the second axis may be perpendicular to each other. Although not illustrated, in another example, the plan view of the finger contact surface 11 may have first and second axes that are equal in length. In addition, in FIG. 2B, the plan view of the finger contact surface 11 is illustrated as a rounded rectangle, but the plan view of the finger contact surface 11 may have other shapes, such as a normal rectangle, a square, an oval, a circle, and the like.

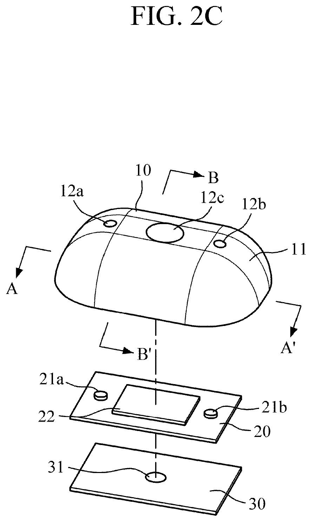

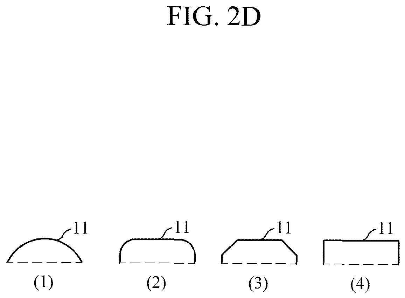

FIG. 2C is an exploded perspective view of the sensor part 10. FIG. 2D illustrates a three-dimensional shape of the finger contact surface 11.

A first-axis (A-A') cross-section of the finger contact surface 11 may be convexly curved in an outward direction of the main body MB. For example, the first-axis cross-section (A-A' cross-section) of the finger contact surface 11 may have a shape in which the height of the cross-section gradually decreases as the distance to the center of the finger contact surface 11 increases, as shown in (1) of FIG. 2D. For example, the first-axis cross-section of the finger contact surface 11 may have the same or similar shape to a portion of a circular or elliptical shape.

In another example, the first-axis cross-section of the finger contact surface 11 may have a shape in which the height of a given region of the cross-section from the center of an upper portion is horizontal and the height gradually decreases thereafter as the distance to the center increases. For example, the first-axis cross-section may have a shape in which the height gradually decreases in the form of a curve, as shown in (2) of FIG. 2D, or in a straight line, as shown in (3), after a given point at a predetermined radius from the center of the upper portion. In this case, the finger contact surface 11 may be gradually lowered in a curved or straight line from the upper portion to a given point and be vertically lowered after the given point to a bottom portion, or may be continuously lowered in a curved or straight line from the upper portion to the bottom portion.

In another example, the first-axis cross-section of the finger contact surface 11 may be a plane. For example, as shown in (4) of FIG. 2D, the first-axis cross-section of the finger contact surface 11 is horizontal and the left and right ends each may have a right-angled shape.

The examples of the shape of the first-axis cross-section of the finger contact surface 11 may also be applied as examples of the shape of a second-axis cross-section (B-B' cross-section). The first-axis cross-section and the second-axis cross-section may have the same shape or different shapes. For example, the first-axis cross-section and the second-axis cross-section may both have the same shape as (2) of FIG. 2D, or the first-axis cross-section may have the same shape as (3) of FIG. 2D and the second-axis cross-section may have the same shape as (2) of FIG. 2D. For example, in the exemplary embodiment of FIG. 2D, the first axis and the second axis of the finger contact surface 11 may each be arranged similarly to (2) of FIG. 2D and the first axis is longer than the second axis.

When the finger contact surface 11 forms a curved surface, a deeper deformation of the finger may be made, as compared to a case of a flat surface, when the finger is pressed with the same force. Accordingly, it is possible for the user to produce the same deformation of the finger by applying less force to the finger contact surface 11.

Referring back to FIG. 2C, the finger contact surface 11 may include a first light transmissive region 12a formed on one part thereof, a second light transmissive region 12b spaced apart from the first light transmissive region 12a and formed on another part thereof, and a third light transmissive region 12c formed between the first light transmissive region 12a and the second light transmissive region 12b. The remaining region of the finger contact surface 11 may be a non-light-transmissive region. The first light transmissive region 12a, the second light transmissive region 12b, and the third light transmissive region 12c may each include holes formed in a circular, elliptical, or polygonal shape. In addition, each hole may be closed with a cover made of a transparent material, such as glass, plastic, or the like, to pass light therethrough. In this case, an individual cover may be configured to close each hole, or one cover integrally formed may be configured to cover all three holes. The first light transmissive region 12a, the second light transmissive region 12b, and the third light transmissive region 12c may be arranged on the first axis.

The optical sensor 20 may be disposed inside the housing HS. However, the embodiment is not limited thereto, and the optical sensor 20 may be disposed at a lower end outside the housing HS.

The optical sensor 20 may include light sources 21a and 21b that irradiate a finger when the finger is placed on and in contact with the finger contact surface 11, and a photodetector 22 that detects light scattered or reflected by the tissue on the surface or inside of the finger that is irradiated by the light sources 21a and 21b.

The light sources 21a and 21b may include a first light source 21a and a second light source 21b disposed on both sides of a substrate of the optical sensor 20 as illustrated. However, the number of light sources is not limited. In this case, the light sources 21a and 21b may include at least one of a light-emitting diode (LED), a laser diode, and a phosphor, but are not limited thereto.

The first light source 21a and the second light source 21b may be configured to emit light of different wavelengths from each other. For example, both of the first light source 21a and the second light source 21b may emit light of infrared (IR) wavelength band or green wavelength band. Alternatively, one of the first light source 21a and the second light source 21b may emit light of infrared wavelength and the other may emit light of green wavelength. In addition, each of the light sources 21a and 21b may include a plurality of LEDs and the plurality of LEDs may all be configured to emit light of the same wavelength or some of the plurality of LEDs may be configured to emit light of different wavelengths. For example, the light source 21a may include an IR LED which emits light of an infrared wavelength and a green LED which emits light of a green wavelength, and the light source 21b may also include an IR LED and a green LED.

The photodetector 22 may be interposed between the first light source 21a and the second light source 21b on the substrate of the optical sensor 20. The photodetector 22 may be a complementary metal-oxide semiconductor (CMOS) image sensor, but is not limited thereto such that the photodetector 22 may include a photodiode, a phototransistor (PTr), a charge-coupled device (CCD) image sensor, and the like. When the light scattered or reflected by the finger is detected, the photodetector 22 may convert the intensity of the light into electrical digital light signal data and transmit the digital light signal data to a processor.

In addition, the force sensor 30 may be disposed inside of the housing HS or at the bottom outside of the housing HS. The force sensor 30 may be laminated on the bottom or the top of the optical sensor 20. The force sensor 30 may measure a pressing force of a finger in contact with the finger contact surface 11. For example, the force sensor 30 may include a strain gauge, and measure the magnitude of force at which the user presses the sensor 10.

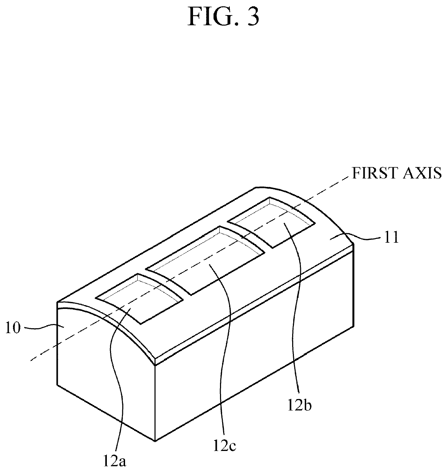

FIG. 3 is a perspective view of a sensor part 10 according to another embodiment. In FIG. 3, a first-axis cross section of a finger contact surface 11 has a similar shape to (3) of FIG. 2D, and a second-axis cross section has a similar shape to (1) of FIG. 2D. The finger contact surface 11 of FIG. 3 includes a first light transmissive region 12a, a second light transmissive region 12b, and a third light transmissive region 12c.

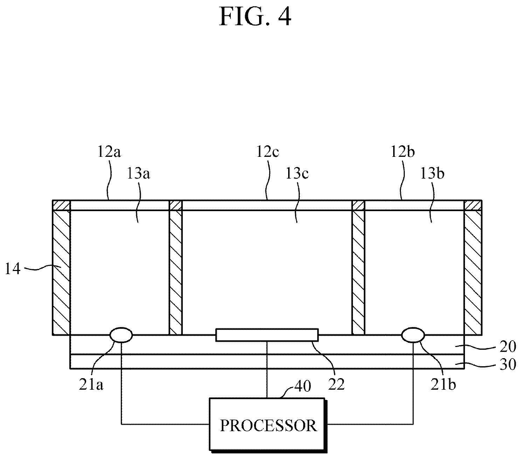

FIG. 4 is a cross-sectional view of the sensor part 10 shown in FIG. 3. Specifically, FIG. 4 is a cross-sectional view of the sensor part 10 of FIG. 3 taken along a first axis of the finger contact surface 11. Referring to FIG. 4, a housing HS may include a first light path 13a and a second light path 13b that guide light emitted by a first light source 21a and a second light source 21b to pass through the first light transmissive region 12a and the second light transmissive region 12b and be directed toward the finger in contact with the finger contact surface 11. In addition, the housing HS may include a third light path 13c that guides light to pass through the third light transmissive region 12c and be directed toward the photodetector 22 when the light, which is emitted by the first light source 21a and the second light source 21b, is scattered or reflected from the surface or internal tissue of the finger in contact with the finger contact surface 11.

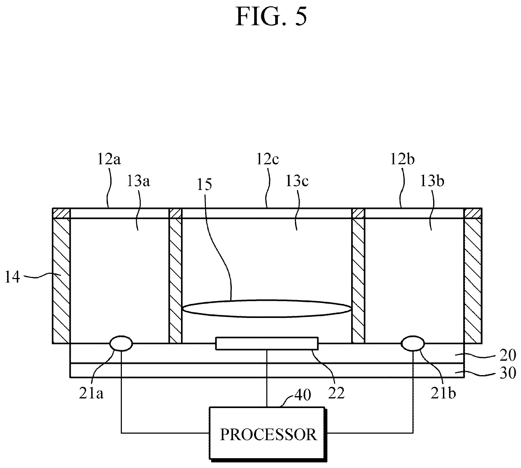

FIG. 5 is a cross-sectional view of a sensor part 10 according to another embodiment. Referring to FIG. 5, a third light path 13c may further include an optical module 15, for example, a lens, to condense light scattered or reflected from a finger toward a photodetector 22. In addition, a filter may be disposed on the third light path 13c to pass light of a predefined wavelength and condense the light to the photodetector 22.

Also, referring to FIGS. 4 and 5, the light paths 13a, 13b, and 13c of the housing HS may be partitioned from each other by partition walls 14. The partition walls 14 may prevent the light emitted from the light sources 21a and 21b from directly entering the photodetector 22. The partition walls 14 may be made of a non-light-transmissive material. The partition walls 14 may be manufactured integrally with the housing HS.

A processor 40 is embedded in the main body MB of the electronic device 100. The processor 40 is electrically connected to the sensor part 10. The processor 40 may control the optical sensor 20 and the force sensor 30, receive measured data from the optical sensor 20 and the force sensor 30, and process the received data.

The processor 40 may control the sensor part 10 to operate in a bio-information measurement mode or in a general mode.

For example, the processor 40 may operate in the general mode, and receive a command that the user inputs by manipulating the sensor part 10, and process the received command. For example, when the user manipulates the sensor part 10 in the general mode or requests execution of a bio-information estimation application through a display DP capable of receiving touch input, an interface related to the bio-information estimation application may be output to the display DP by executing the bio-information estimation application.

When a request for estimating bio-information is received according to manipulation of the sensor part 10 in the general mode or manipulation of the display DP, the processor 40 may switch the mode of the sensor part 10 to the bio-information estimation mode and control the electrically connected light sources 21a and 21b, photodetector 22, and force sensor 30. For example, the processor may control the intensity of light, duration of light, and on/off statuses of the light sources 21a and 21b and power supply to the force sensor 30.

When the processor 40 receives light signal data from the photodetector 22 and contact force data from the force sensor 30 in the bio-information estimation mode, the processor 40 may process the light signal data and the contact force data by executing, for example, a predefined bio-information estimation algorithm. For example, the processor 40 may monitor an environment for measuring bio-signal by using the light signal data and/or the contact force data, thereby guiding the user to maintain a normal measurement environment, and may estimate bio-information using a measured bio-signal.

The processor 40 may output a data processing result using various output modules of the electronic device 100, for example, the display DP, a speaker, and the like. The processor 40 may visually display various graphical objects that guide the environment for measuring a bio-signal on the display DP, in which case the various graphical objects may be provided as a gamified screen or in the form of various graphs, so as to intuitively arouse a user's interest.

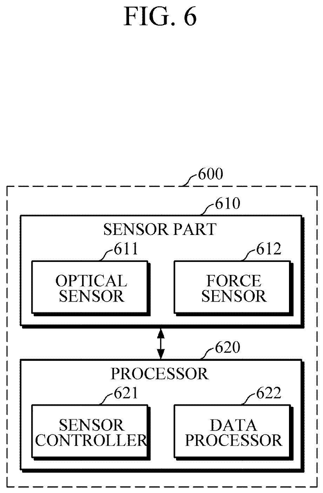

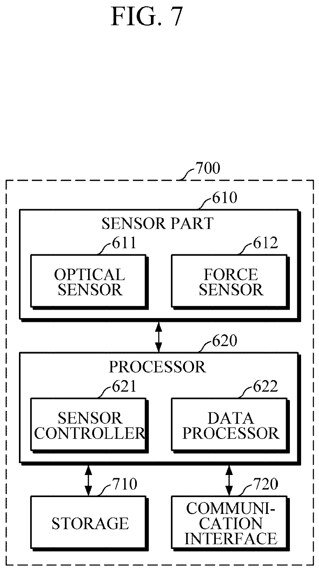

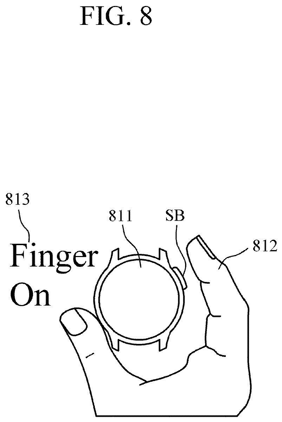

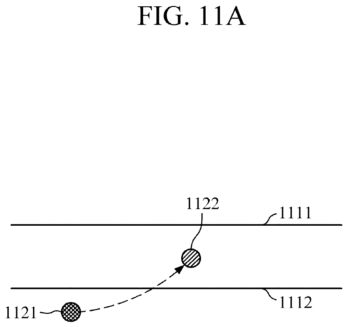

FIGS. 6 and 7 are block diagrams illustrating an electronic device according to exemplary embodiments. FIGS. 8 to 10 are diagrams illustrating exemplary embodiments in which a graphical object related to a contact state is displayed. FIGS. 11A to 12B are diagrams illustrating exemplary embodiments in which a graphical object related to a contact force is displayed.

Referring to FIG. 6, an electronic device 600 includes a main body provided in various shapes, and a sensor part 610 and a processor, which are disposed in the main body. In this case, the main body may be in the form of a smart watch as described with reference to FIG. 1, but is not limited thereto, and may be provided in the form of a mobile device, such as smartphone or a tablet PC.

The sensor part 610 includes an optical sensor 611 and a force sensor 612.

The optical sensor 611 may include one or more light sources configured to emit light to a finger when the finger comes in contact with a finger contact surface, and a photodetector configured to detect light scattered or reflected from the surface and/or internal tissue of the finger irradiated by the light sources. The one or more light sources may emit light of different wavelengths from each other.

The force sensor 612 may measure a contact force applied by the finger in contact with the finger contact surface to the finger contact surface.

The processor 620 may include a sensor controller 621 and a data processor 622.

The sensor controller 621 may control the sensor part 610 to operate in a general mode or in a bio-information estimation mode. For example, when the electronic device 600 is driven, the sensor controller 621 may control the sensor part 610 in the general mode. When the user manipulates a button of the sensor part 610 in the general mode or requests estimation of bio-information by performing an action, such as touch/drag of a display, the mode of the sensor part 610 may be switched to the bio-information estimation mode. In addition, when the user inputs a predefined gesture through a camera module mounted in the electronic device 100 or inputs a voice command through a microphone mounted in the electronic device 100, the mode of the sensor part 610 may be switched to the bio-information estimation mode.

When a request for estimating bio-information is received and accordingly the mode of the sensor part 610 is switched to the bio-information estimation mode, the sensor controller 621 may, for example, drive the light sources of the optical sensor 611 and control the intensity of electric current, duration, or the like, of the light sources. Also, the sensor controller 621 may supply power to various modules including the force sensor 612.

When the sensor part 610 measures a bio-signal, the data processor 622 may monitor a measurement and guide the user to normally measure the bio-signal.

For example, when the sensor controller 621 switches the mode of the sensor part 610 to the bio-information estimation mode as described above, the data processor 622 displays a graphical object to induce the user to bring his/her finger properly into contact with the finger contact surface. Here, the graphical object may include, but is not limited to, text, characters, icons, images, figures, and the like.

Referring to FIG. 8, the data processor 622 displays a first graphical object 811 representing the appearance of the main body on the display. In addition, the data processor 662 also displays a second graphical object 812 representing the appearance of the finger on the display to induce the user to properly touch a position of the sensor part SB with a measurement site of the finger, for example, the tip of the finger. In this case, text 813 may be displayed to induce the contact of the finger.

For example, the data processor 622 may simultaneously display the first graphical object 811 and the second graphical object 812 on the display by generating one graphical image including the first graphical object 811 and the second graphical object 812. In another example, the second graphical object 812 and/or the text 813 may repeatedly appear and disappear once or more at specific time intervals after a predetermined period of time while the first graphical object 811 is being displayed.

In addition, when the user touches the finger contact surface of the sensor part 610 with the finger according to the guidance and accordingly contact state information is received from the optical sensor 611, the data processor 622 determines whether the contact state of the finger is normal. For example, based on the intensity of light signal received from the optical sensor 611, image data, fingerprint data, and the like, the data processor 622 may determine the contact state, such as whether the finger is in contact, the contact position, an initial contact force of the finger received from the force sensor 612, or the like.

For example, when a predefined criterion is satisfied, for example, when the force sensor 612 measures a contact force that is greater than or equal to a predefined threshold value, measures a contact force for a period greater than or equal to a threshold period, or measures a contact force greater than or equal to the predefined threshold value for a period greater than or equal to a threshold period, the data processor 622 may determine that the contact state is normal. However, the embodiment is not limited thereto.

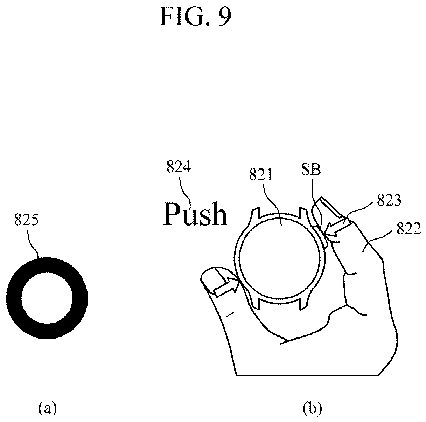

When it is determined that the contact state is normal, the data processor 622 displays a graphical object on the display to induce the measurement of a bio-signal. As shown in FIG. 9, the first graphical object 821 representing the appearance of the main body and the second graphical object 822 representing the appearance of the finger that is in normal contact with the main body are displayed on the display. In this case, a sensor part SB in the first graphical object 821 may be emphasized in a different color.

In addition, a third graphical object 823 is displayed to induce the user to exert a force with the finger toward the sensor part SB. In this case, the third graphical object 823 may include an arrow superimposed on the second graphical object 822 as illustrated. Moreover, in another example, the third graphical object 823 may be modified from the second graphical object 822 such that an index finger repeatedly moves from a predetermined position spaced apart from the sensor part SB to the sensor part SB.

In addition, as shown in FIG. 9, the processor 620 may display a fourth graphical object 825 indicating that the contact state is normal and/or text 824 requesting an action of pressing with a finger.

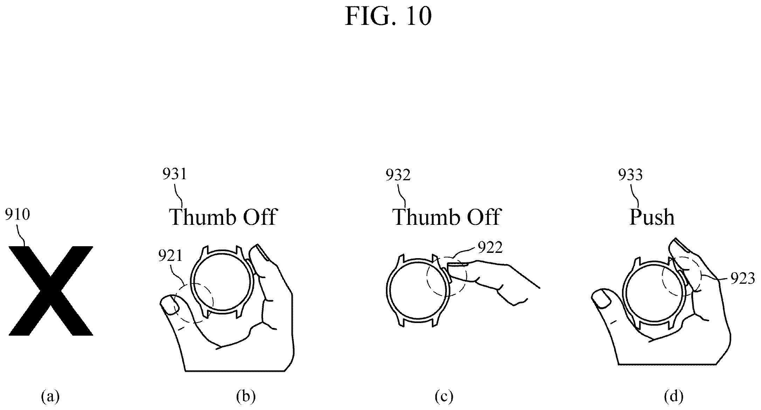

When it is determined that the contact state is abnormal, the data processor 622 may display a graphical object to induce the user to bring his/her finger again into contact with the finger contact surface. For example, as shown in FIG. 10, a fifth graphical object 910 indicating that the contact state is abnormal (a) may be displayed. In addition, for example, (b) when a thumb is not in contact normally, (c) when an initial contact force falls out of a normal threshold, and (d) when a normal measurement site of an index finger (e.g., the tip of the index finger) is not in contact, texts 931, 932, and 933 each explaining the reason for being non-normal and/or sixth graphical objects 921, 922, and 923 each visually indicating the reason for being non-normal may be displayed.

When it is determined that the contact state is normal, the data processor 622 guides a contact force so that the user presses the sensor part 610 with his/her finger at an appropriate force while a bio-signal is being measured.

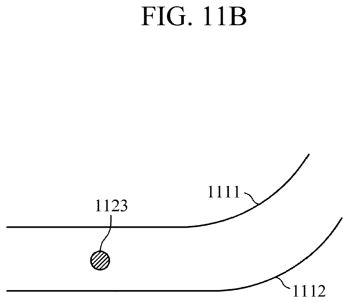

For example, FIG. 11A illustrates a screen in a measurement initial state in which an initial contact force is adjusted with a finger, and FIG. 11B illustrates a screen after the initial contact force falls within a normal range.

Referring to FIGS. 11A and 11B, the data processor 622 may display seventh graphical objects 1111 and 1112 representing predefined reference contact forces and eighth graphical objects 1121, 1122, and 1123 representing an actual contact force received from the force sensor 612. For example, as illustrated, the upper limit 1111 and the lower limit 1112 of the seventh graphical objects may include lines, continuous points, circles, ellipses, polygons, and the like. Similarly, the eighth graphical objects 1121, 1122, and 1123 may include lines, continuous points, circles, ellipses, polygons, and the like.

Referring to FIG. 11A, the data processor 622 may display the upper limit 1111 and the lower limit 1112 of the seventh graphical objects on a display screen in a horizontal direction at an initial stage of measurement. In addition, when the initial contact force received from the force sensor 612 falls out of the upper limit and/or the lower limit of the reference contact force, for example, when the initial contact force is not measured or is less than the lower limit of the reference contact force as illustrated, the eighth graphical object 1121 may be displayed below the lower limit object 1112. Also, when the actual contact force falls within the normal range of the reference contact force as the user adjusts a force of a finger pressing the sensor part 610, the eighth graphical object 1122 may be displayed at a position between the upper limit object 111 and the lower limit object 1112, corresponding to the actual contact force. In this case, the eighth graphical objects 1121 and 1122 may be displayed as if moving, so that the change in the actual contact force is displayed. Further, the eighth graphical object 1121 out of the normal range and the eighth graphical object 1122 within the normal range may be distinguished from each other by different shapes or colors, so as to be easily recognizable by the user. For example, the eighth graphical object 1121 out of the normal range may be displayed, for example, in red, and the eighth graphical object 1122 within the normal range may be displayed, for example, in green.

Referring to FIG. 11B, when the actual contact force falls within the normal range at the initial stage of measurement, the data processor 622 may change the shapes of the seventh graphical objects 1111 and 1112 horizontally arranged as shown in FIG. 11A to gradual upward sloping curves, such that the user gradually increases the pressing force of the finger over time. In this way, the seventh graphical objects 1111 and 1112 may be changed to upward sloping shapes or downward sloping shapes according to the change in the reference contact force for a period when the bio-signal is measured.

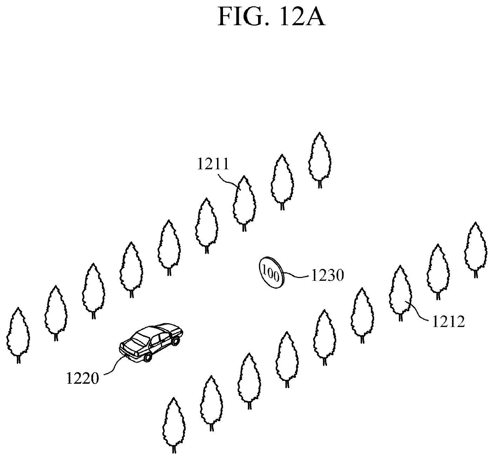

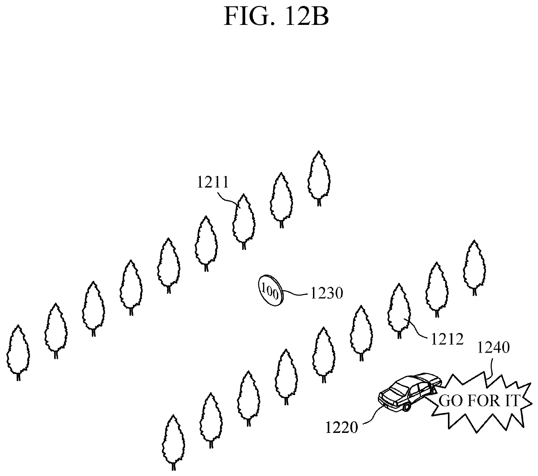

Referring to FIGS. 12A and 12B, the data processor 622 may construct a game screen and display the game screen on the display to arouse the user's interest and to guide the user to maintain the contact force within the normal range for the period of measurement while checking the contact force easily and intuitively. For example, as illustrated, seventh graphical objects 1211 and 1212 may be formed as characters of "street trees" so that a space between the upper limit 1211 and the lower limit 1212 of the reference contact force represents a "tree-lined road." However, the exemplary embodiment is not limited thereto, such that types of characters representing the upper limit and the lower limit and the game screen are not particularly limited. In addition, the eighth graphical object 1220 may be a game character, such as a "car," "bird," "animal," or the like, which moves along the "tree-lined road" in response to the actual contact force received in real-time from the force sensor 612. However, the type of the game character is not particularly limited.

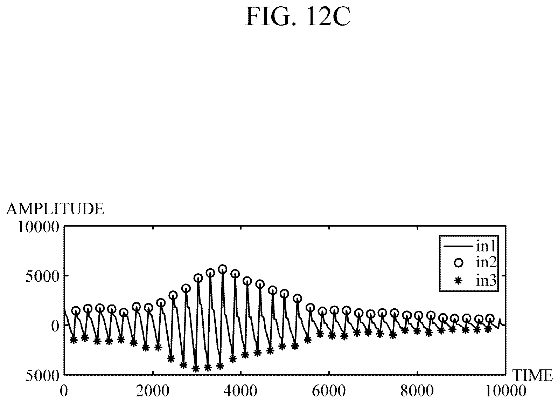

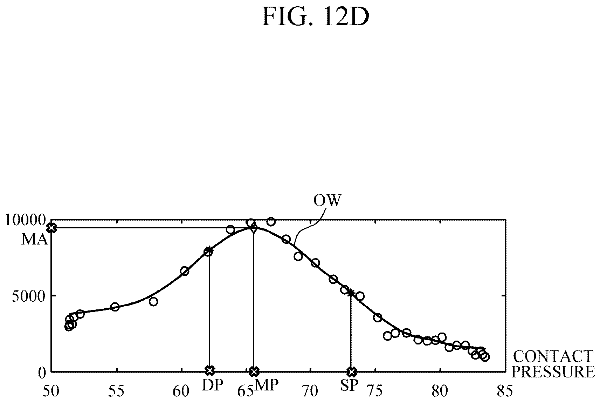

For example, the eighth graphical object 1220 may move upward in the game screen as the actual contact force received from the force sensor 612 increases, and may move downward in the game screen as the actual contact force decreases. In this case, the seventh graphical object may include a game item 1230 to provide the user with a benefit, such as a game score, when the actual contact force received from the force sensor 612 is maintained normally for a predetermined period of time. In addition, when the actual contact force received from the force sensor 612 falls out of the reference contact force, the data processor 622 may display a ninth graphical object 1240 on the display to give a visual warning as shown in FIG. 12. However, the embodiment is not limited thereto, and a warning sound may be output through a voice output module, such as a speaker.

In the foregoing description, the examples in which the data processor 622 sequentially displays the graphical objects of FIG. 8, the graphical objects of FIG. 9, and the graphical objects of FIG. 11 (or FIG. 12) on the display according to the request for estimating bio-information and the measurement environment are described. However, the present disclosure is not limited thereto, and at least some of the graphical objects of FIG. 9, the graphical objects of FIG. 9, the graphical objects of FIG. 10, and the graphical objects of FIG. 11 (or FIG. 12) may be omitted. The invention remains however defined by the appended claims.

For example, when a request for estimating bio-information is received, the graphical objects of FIG. 8 may be displayed, and when the contact state is normal, the graphical objects of FIG. 11 (or FIG. 12) may be immediately displayed. Alternatively, when the contact state is determined without displaying the graphical objects of FIG. 8 and the determination result shows that the contact state is normal, the graphical objects of FIG. 9 may be immediately displayed and then the graphical objects of FIG. 11 (or FIG. 12) may be displayed. Alternatively, when the graphical objects of FIGS. 8 and 9 are not displayed and it is determined that the contact state is normal, the graphical objects of FIG. 11 (or FIG. 12) may be immediately displayed. Alternatively, when the graphical objects of FIG. 8 are not displayed and the contact state is not normal, the graphical objects of FIG. 10 may be displayed, and when the contact state becomes normal, the graphical objects of FIG. 11 (or FIG. 12) may be immediately displayed.

The data processor 622 may receive a bio-signal from the optical sensor 611 and preprocess the received bio-signal. In this case, the bio-signal may include a photoplethysmogram (PPG) signal, an impedance plethysmogram (IPG) signal, a pressure wave signal, a video plethysmogram (VPG) signal, and the like. For example, when the biosignal is received, the data processor 622 may remove noise by performing, for example, bandpass filtering at 0.4-10 Hz. Alternatively, bio-signal correction may be performed through fast Fourier transform-based reconstruction of the bio-signal. However, the embodiment is not limited thereto.

In addition, the data processor 622 may estimate bio-information on the basis of data received from the optical sensor 611 and the force sensor 612 by performing an algorithm for estimating bio-information. In this case, the bio-information may include, but is not limited to, mean blood pressure, systolic blood pressure, diastolic blood pressure, a vascular age, arterial stiffness, an aortic artery pressure waveform, a vascular elasticity, a stress index, and a fatigue level.

For example, the data processor 622 may extract features including a peak amplitude value, a time of a peak amplitude point, pulse waveform components, the area of a predetermined section of a bio-signal, and the like, from the bio-signal, and estimate the bio-information by combining one or more extracted features. In this case, the bio-information may be estimated by using a predefined bio-information estimation model. The bio-information estimation model may be defined as various linear or non-linear combination functions, such as addition, subtraction, division, multiplication, logarithmic value, regression equation, and the like, with no specific limitation.

In another example, the data processor 622 may acquire a contact pressure on the basis of the contact force received from the force sensor and the area of the finger contact surface of the sensor part 610, and estimate a blood pressure on the basis of oscillometry based on a maximum peak point of the bio-signal and the contact pressure.

FIG. 12C illustrates an intensity of bio-signal measured by gradually increasing force in a state where the user touches the finger contact surface with an object. FIG. 12D illustrates an oscillometric envelope acquired based on an intensity of bio-signal and a contact pressure. For example, the data processor 622 may extract a peak-to-peak point by subtracting an amplitude value in3 at a minus (-) point from an amplitude value in2 at a plus (+) point of a waveform envelope in1 at each measurement point in time and acquire the oscillometric envelope OW by plotting the amplitude of the extracted peak-to-peak point at each measurement point in time based on a contact pressure at the corresponding measurement time point.

The data processor 622 may acquire features for estimating blood pressure from the acquired oscillometric envelope OW. For example, the features for estimating blood pressure may include an amplitude value MA at a maximum peak point, a contact pressure value MP at the maximum peak point, contact pressure values SP and DP that are on the left and right sides and predetermined proportions (e.g., 0.5 to 0.7) of the contact pressure value MP at the maximum peak point. When the features are acquired, the data processor 622 may estimate blood pressure by applying the features to a predefined blood pressure estimation model.

Referring to FIG. 7, the electronic device 700 may include a sensor part 610, a processor 620, a storage 719, and a communication interface 720. The sensor part 610 may include an optical sensor 611 and a force sensor 612, and the processor 620 may include a sensor controller 621 and a data processor 622. The sensor part 610 and the processor 620 are described in detail with reference to FIG. 6, and hence the following description will be made based on details that are not redundant.

The data processor 622 displays graphical objects related to a contact state, a contact force, and the like, through a display as described above. In addition, when a bio-information estimate value is obtained, the data processor 622 may visually display the obtained bio-information estimate value through the display. In this case, when a bio-information estimation result falls out of a normal range, the data processor 622 may visually output alarm/warning information. Alternatively, the data processor 622 may non-visually output warning information related to a contact state, a contact force, and a bio-information estimate value in voice or through a non-visual output means, such as a haptic device.

The storage 710 may store reference information for estimating bio-information and a processing result of the sensor part 610 and/or the processor 620. In this case, the reference information may include user information, such as a user's age, gender, health condition, and the like, a normal contact state, such as a contact position of a finger or the like, a condition for driving a light source, a reference contact force, a bio-information estimation model, and the like. However, the reference information is not limited to these examples.

The storage 710 may include at least one type of storage medium of a flash memory type, a hard disk type, a multimedia card micro type, a card type memory (for example, secure digital (SD) or extreme digital (XD) memory), a random access memory (RAM), a static random access memory (SRAM), a read-only memory (ROM), an electrically erasable programmable read-only memory (EEPROM), a programmable read-only memory (PROM), a magnetic memory, a magnetic disk, and an optical disk, but is not limited thereto.

The communication interface 720 may communicate with an external device under the control of the processor 620 and transmit and receive various types of data related to bio-information estimation. For example, the communication interface 729 may transmit the processing result of the processor 620 to the external device, so that the external device performs management of bio-information history for the user, monitoring of the user's health condition, output of bio-information history and the health condition monitoring result, and the like. In this case, the external device may include, but not limited to, a smartphone, a tablet PC, a desktop computer, a notebook computer, and devices of a medical institution including a cuff-based blood pressure measurement device.

In another example, the communication interface 720 may receive a bio-information estimation model required for estimating bio-information, characteristic information of a user, and the like from the external device. The received information may be stored in the storage 710.

The communication interface 720 may communicate with the external device by using Bluetooth communication, Bluetooth low energy (BLE) communication, near field communication (NFC), wireless local access network (WLAN) communication, ZigBee communication, infrared data association (IrDA) communication, wireless fidelity (Wi-Fi) Direct (WFD) communication, ultra-wideband (UWB) communication, Ant+ communication, Wi-Fi communication, radio frequency identification (RFID) communication, 3G communication, 4G communication, and/or 5G communication. However, these are merely examples, and the embodiment is not limited thereto.

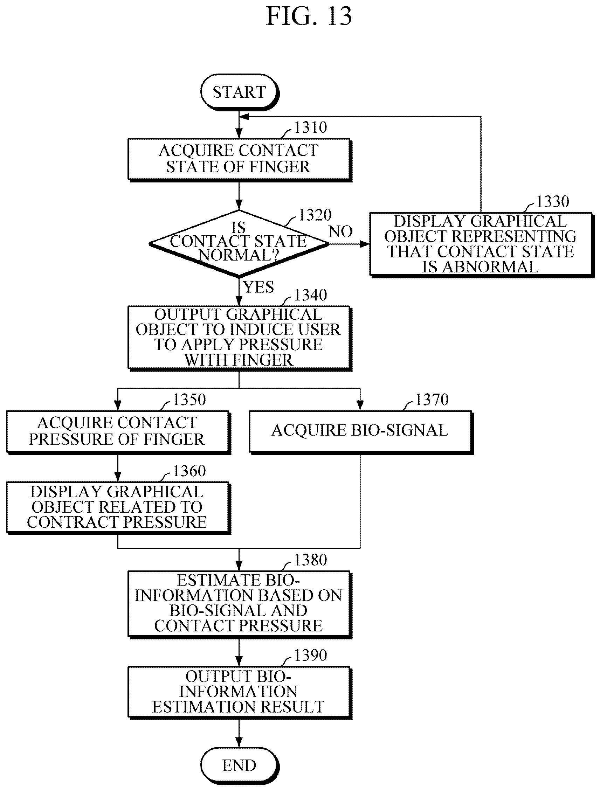

FIG. 13 is a flowchart illustrating a method of estimating bio-information according to an exemplary embodiment.

The method of FIG. 13 may be one embodiment of a bio-information estimating method performed by the electronic device 100/600/700 according to the above-described embodiments. Hereinafter, the method will be described in brief to avoid redundancy.

First, when a finger is in contact with the sensor part, the electronic device acquires a contact state of the finger through the sensor part (operation 1310). Upon receiving a request for estimating bio-information, the electronic device displays a graphical object on a display to guide the finger to be in contact normally. The graphical object includes a graphical object representing the appearance of the main body and a graphical object representing the appearance of the finger to guide the finger to be in normal contact with the sensor part.

Then, based on information on a contact state of the finger acquired in operation 1310, it is determined whether or not the contact state is normal (operation 1320). For example, whether the contact state is normal or abnormal may be determined by analyzing whether an accurate measurement site of the finger is in contact with the sensor part, whether an initial contact force is within a preset threshold range, whether a contact force is measured for a threshold period of time, or the like.

Then, when it is determined that the contact state is abnormal (operation 1320 - NO), a graphical object indicating that the contact state is abnormal may be displayed in order to guide the contact state to the normal state (operation 1330).

When it is determined that the contact state is normal (operation 1320 - YES), a graphical object is displayed to induce the user to apply a pressure to the sensor part with a finger for measuring a bio-signal (operation 1340). For example, a graphical object representing a finger may repeatedly blink at a position of the sensor part or an arrow may be displayed, in order to emphasize the contact of the finger with the sensor part for the sake of easy recognition by the user.

Then, when a contact force of the finger is acquired (operation 1350), at the same time, a graphical object related to the contact force is displayed to induce the user to press the sensor part with a predefined normal force (operation 1360). A graphical object related to a reference contact force at which the finger must press the sensor part and a graphical object representing an actual contact force measured through the force sensor are displayed. In this case, the graphical objects may be presented as a gamified screen to induce the user to maintain the contact force while arousing the user's interest.

In addition, a bio-signal may be obtained through the sensor part while the user changes the contact force with the finger according to the guidance for the contact force (operation 1370).

Then, bio-information is estimated based on the bio-signal and the contact force (operation 1380). For example, as described above, a feature of a maximum peak point may be extracted from the bio-signal, and blood pressure may be estimated using the extracted feature and a bio-information estimation model, or a contact pressure may be obtained based on the contact force and blood pressure may be estimated based on oscillometry.

Then, a bio-information estimation result may be output (operation 1390). The bio-information estimation result may be visually output to the display, or non-visually output by using a speaker, a haptic module, or the like.

The example embodiments can be implemented as computer-readable code stored in a non-transitory computer-readable medium. Code and code segments constituting the computer program can be inferred by a computer programmer skilled in the art. The non-transitory computer-readable medium includes all types of record media in which computer-readable data are stored.

Examples of the non-transitory computer-readable medium include a ROM, a RAM, a CD-ROM, a magnetic tape, a floppy disk, and an optical data storage. Further, the record medium may be implemented in the form of a carrier wave such as Internet transmission. In addition, the non-transitory computer-readable medium may be distributed to computer systems over a network, in which computer readable codes may be stored and executed in a distributed manner.

A number of examples have been described above. Nevertheless, it will be understood that various modifications may be made. For example, suitable results may be achieved if the described techniques are performed in a different order and/or if components in a described system, architecture, device, or circuit are combined in a different manner and/or replaced or supplemented by other components.

The invention is defined by the appended claims.

## Claims

1. A method of estimating bio-information which is performed by an electronic device (600; 700) comprising a sensor part (610) disposed on a side of a main body (MB) with a display (DP), and a processor (620), the method comprising:
receiving a request for estimating bio-information;
controlling the display to display a first graphical object (811) representing an appearance of the main body including a sensor part (SB) and a second graphical object (812) representing an appearance of a finger to guide the finger to be in normal contact with the sensor part (SB);
acquiring (1310), by the sensor part (610), a contact state of a finger before measurement of a bio-signal, wherein the sensor part comprises an optical sensor (611) and a force sensor (612) configured to measure the contact force based on the finger contacting the finger contact surface;
determining (1320) whether the contact state of the finger is normal based on at least one of an analysis whether an initial contact force is within a preset threshold range, and an analysis whether a contact force is measured for a threshold period of time;
in response to the contact state being normal, controlling a display, by the processor (620), to display the first graphical object (821), the second graphical object (822), and a third graphical object (823) to induce the user to apply a pressure to the sensor part (SB) with the finger for measuring the bio-signal;
acquiring (1350), by the sensor part (610), a contact force of the finger during the measurement of the bio-signal;
controlling (1360) the display, by the processor (620), to display a seventh graphical object (1111, 1112) related to a reference contact force at which the finger presses the sensor part (SB) and an eighth graphical object (1121, 1122) representing an actual contact force measured through the force sensor to induce the user to press the sensor part (SB) with a predefined normal force;
acquiring (1370) the bio-signal by the sensor part (SB); and
estimating bio-information (1380) based on the bio-signal and the contact force.

2. The method of claim 1 further comprising:
in response to the contact state being abnormal, controlling the display to display at least one of a fifth graphical object (910) indicating that the contact state is abnormal, a sixth graphical object (921, 922, 923) visually displaying a reason for the contact state being abnormal, and text (931, 932, 933) describing a reason for the contact state being abnormal.

3. An electronic device (600; 700) comprising a main body (MB) with a display (DP), a sensor part (610) disposed on a side of the main body and a processor (620), wherein the electronic device (600; 700) is configured to perform the method of one of claims 1 to 2.

4. A non-transitory computer-readable storage medium storing instructions that, when
executed by a processor (620) of an electronic device (600; 700) comprising a main body (MB) with a display (DP),
a sensor part (610) disposed on a side of the main body, and the processor (620), cause the processor (620) to:
receive a request for estimating bio-information;
control the display to display a first graphical object (811) representing an appearance of the main body including a sensor part (SB) and a second graphical object (812) representing an appearance of a finger to guide the finger to be in normal contact with the sensor part (SB);
acquire, by the sensor part (610), a contact state of a finger before measurement of a biosignal;
determine whether the contact state of the finger is normal based on at least one of an analysis whether an initial contact force is within a preset threshold range, and an analysis whether a contact force is measured for a threshold period of time;
In response to the contact state is normal, control a display, by the processor (620), to display the first graphical object (821), the second graphical object (822), and a third graphical object (823) to induce the user to apply a pressure to the sensor part (SB) with the finger for measuring the bio-signal;
acquire, by the sensor part (610), a contact force of the finger during the measurement of the bio-signal;
control the display, by the processor (620), to display a seventh graphical object (1111, 1112) related to a reference contact force at which the finger presses the sensor part (SB) and a eighth graphical object (1121, 1122) representing an actual contact force measured through the force sensor to induce the user to press the sensor part (SB) with a predefined normal force;
acquire the bio-signal by the sensor part (SB); and
estimate bio-information based on the bio-signal and the contact force,
wherein the sensor part (610) comprises an optical sensor (611) comprising a light source configured to emit light to the finger in contact with a finger contact surface and a photodetector configured to detect light scattered or reflected from the finger, and a force sensor (612) configured to measure the contact force based on the finger contacting the finger contact surface.

5. The computer-readable storage medium of claim 4,
wherein the processor is further configured to control the display to display the second graphical object (812) or a text (813) to repeatedly appear and disappear once or more at specific time intervals after a predetermined period of time while the first graphical object (811) is being displayed.

6. The computer-readable storage medium of claim 5, wherein the third graphical object (823) represents an appearance of the finger that repeatedly moves from a predetermined position spaced apart from the sensor part (SB) of the main body to a position of the sensor part (SB), or wherein the third graphical object (823) includes an arrow directed toward the sensor part (SB).

7. The computer-readable storage medium of claim 6, wherein the processor is further configured to control the display to display a fourth graphical object (825) indicating that the contact state is normal based on the contact state being normal, or
wherein the processor is further configured to display at least one of a fifth graphical object (910) indicating that the contact state is abnormal, a sixth graphical object (921, 922, 923) visually displaying a reason for the contact state being abnormal, and text (931, 932, 933) describing a reason for the contact state being abnormal based on the contact state being abnormal.

## Patentansprüche

1. Verfahren zum Schätzen von Bioinformationen, das von einer elektronischen Vorrichtung (600; 700) durchgeführt wird, umfassend einen Sensorteil (610), angeordnet auf einer Seite eines Hauptkörpers (MB) mit einer Anzeige (DP) und einem Prozessor (620), wobei das Verfahren umfasst:
Empfangen einer Anforderung zum Schätzen von Bioinformationen;
Steuern der Anzeige zum Anzeigen eines ersten grafischen Objekts (811), das ein Erscheinungsbild des Hauptkörpers umfassend einen Sensorteil (SB) darstellt, und eines zweiten grafischen Objekts (812), das ein Erscheinungsbild eines Fingers darstellt, um den Finger so zu führen, dass er in normalem Kontakt mit dem Sensorteil (SB) steht;
Erfassen (1310) eines Kontaktzustands eines Fingers durch den Sensorteil (610) vor der Messung eines Biosignals, wobei der Sensorteil einen optischen Sensor (611) und einen Kraftsensor (612), ausgebildet zum Messen der Kontaktkraft basierend auf dem Finger, der die Fingerkontaktfläche berührt, umfasst;
Bestimmen (1320), ob der Kontaktzustand des Fingers normal ist, basierend auf wenigstens einer Analyse, ob eine anfängliche Kontaktkraft innerhalb eines festgelegten Schwellenbereichs liegt, und einer Analyse, ob eine Kontaktkraft für einen Schwellenzeitraum gemessen wird;
als Reaktion darauf, dass der Kontaktzustand normal ist, Steuern einer Anzeige durch den Prozessor (620), um das erste grafische Objekt (821), das zweite grafische Objekt (822) und ein drittes grafisches Objekt (823) anzuzeigen, um den Benutzer zu veranlassen, mit dem Finger einen Druck auf den Sensorteil (SB) auszuüben, um das Biosignal zu messen;
Erfassen (1350) einer Kontaktkraft des Fingers während der Messung des Biosignals durch den Sensorteil (610);
Steuern (1360) der Anzeige durch den Prozessor (620), um ein siebtes grafisches Objekt (1111, 1112), das sich auf eine Referenzkontaktkraft bezieht, mit welcher der Finger auf den Sensorteil (SB) drückt, und ein achtes grafisches Objekt (1121, 1122), das eine tatsächliche Kontaktkraft darstellt, die durch den Kraftsensor gemessen wird, um den Benutzer zu veranlassen, auf den Sensorteil (SB) mit einer vordefinierten Normalkraft zu drücken, anzuzeigen;
Erfassen (1370) des Biosignals durch den Sensorteil (SB); und
Schätzen von Bioinformationen (1380) basierend auf dem Biosignal und der Kontaktkraft.

2. Verfahren nach Anspruch 1, ferner umfassend:
als Reaktion darauf, dass der Kontaktzustand anormal ist, Steuern der Anzeige, um wenigstens ein Element einer Gruppe umfassend ein fünftes grafisches Objekt (910), das angibt, dass der Kontaktzustand anormal ist, ein sechstes grafisches Objekt (921, 922, 923), das visuell einen Grund dafür anzeigt, dass der Kontaktzustand anormal ist, und Text (931, 932, 933), der einen Grund dafür beschreibt, dass der Kontaktzustand anormal ist, anzuzeigen.

3. Elektronische Vorrichtung (600; 700), umfassend einen Hauptkörper (MB) mit einer Anzeige (DP), einem Sensorteil (610), angeordnet auf einer Seite des Hauptkörpers, und einen Prozessor (620), wobei die elektronische Vorrichtung (600; 700) zum Ausführen des Verfahrens nach einem der Ansprüche 1 bis 2 ausgebildet ist.

4. Nichtflüchtiges computerlesbares Speichermedium zum Speichern von Befehlen, die, wenn von einem Prozessor (620) einer elektronischen Vorrichtung (600; 700), umfassend einen Hauptkörper (MB) mit einer Anzeige (DP), einem Sensorteil (610), angeordnet an einer Seite des Hauptkörpers, und dem Prozessor (620), ausgeführt, den Prozessor (620) veranlassen zum:
Empfangen einer Anforderung zum Schätzen von Bioinformationen;
Steuern der Anzeige zum Anzeigen eines ersten grafischen Objekts (811), das ein Erscheinungsbild des Hauptkörpers umfassend einen Sensorteil (SB) darstellt, und eines zweiten grafischen Objekts (812), das ein Erscheinungsbild eines Fingers darstellt, um den Finger so zu führen, dass er in normalem Kontakt mit dem Sensorteil (SB) steht;
Erfassen eines Kontaktzustands eines Fingers vor der Messung eines Biosignals durch den Sensorteil (610);
Bestimmen, ob der Kontaktzustand des Fingers normal ist, basierend auf wenigstens einer Analyse, ob eine anfängliche Kontaktkraft innerhalb eines festgelegten Schwellenbereichs liegt, und einer Analyse, ob eine Kontaktkraft für einen Schwellenzeitraum gemessen wird;
als Reaktion darauf, dass der Kontaktzustand normal ist, Steuern einer Anzeige durch den Prozessor (620), um das erste grafische Objekt (821), das zweite grafische Objekt (822) und ein drittes grafisches Objekt (823) anzuzeigen, um den Benutzer zu veranlassen, mit dem Finger einen Druck auf den Sensorteil (SB) auszuüben, um das Biosignal zu messen;
Erfassen einer Kontaktkraft des Fingers während der Messung des Biosignals durch den Sensorteil (610);
Steuern der Anzeige durch den Prozessor (620), um ein siebtes grafisches Objekt (1111, 1112), das sich auf eine Referenzkontaktkraft bezieht, mit welcher der Finger auf den Sensorteil (SB) drückt, und ein achtes grafisches Objekt (1121, 1122), das eine tatsächliche Kontaktkraft darstellt, die durch den Kraftsensor gemessen wird, um den Benutzer zu veranlassen, auf den Sensorteil (SB) mit einer vordefinierten Normalkraft zu drücken, anzuzeigen;
Erfassen des Biosignals durch den Sensorteil (SB); und
Schätzen von Bioinformationen basierend auf dem Biosignal und der Kontaktkraft,
wobei der Sensorteil (610) einen optischen Sensor (611), umfassend eine Lichtquelle, ausgebildet zum Ausstrahlen von Licht zum Finger in Kontakt mit einer Fingerkontaktfläche, und einen Fotodetektor, ausgebildet zum Erfassen von vom Finger gestreuten oder reflektierten Licht, und einen Kraftsensor (612), ausgebildet zum Messen der Kontaktkraft basierend auf dem Finger, der die Fingerkontaktfläche berührt, umfasst.

5. Computerlesbares Speichermedium nach Anspruch 4,
wobei der Prozessor ferner zum Steuern der Anzeige ausgebildet ist, so dass das zweite grafische Objekt (812) oder ein Text (813) nach einem vorbestimmten Zeitraum, während dem das erste grafische Objekt (811) angezeigt wird, in bestimmten Zeitintervallen wiederholt einmal oder mehrmals erscheint und wieder verschwindet.

6. Computerlesbares Speichermedium nach Anspruch 5, wobei das dritte grafische Objekt (823) ein Erscheinungsbild des Fingers darstellt, der sich wiederholt von einer vorbestimmten, vom Sensorteil (SB) des Hauptkörpers beabstandeten Position zu einer Position des Sensorteils (SB) bewegt, oder wobei das dritte grafische Objekt (823) einen auf den Sensorteil (SB) gerichteten Pfeil umfasst.

7. Computerlesbares Speichermedium nach Anspruch 6, wobei der Prozessor ferner zum Steuern der Anzeige ausgebildet ist, um ein viertes grafisches Objekt (825) anzuzeigen, das angibt, dass der Kontaktzustand normal ist, basierend darauf, dass der Kontaktzustand normal ist, oder
wobei der Prozessor zum Anzeigen von wenigstens einem Element einer Gruppe umfassend ein fünftes grafisches Objekt (910), das angibt, dass der Kontaktzustand anormal ist, ein sechstes grafisches Objekt (921, 922, 923), das visuell einen Grund dafür anzeigt, dass der Kontaktzustand anormal ist, und Text (931, 932, 933), der einen Grund dafür beschreibt, dass der Kontaktzustand anormal ist, basierend darauf, dass der Kontaktzustand anormal ist, ausgebildet ist.

## Revendications

1. Procédé d'estimation de bio-informations qui est effectué par un dispositif électronique (600 ; 700) comprenant une partie capteur (610) disposée sur un côté d'un corps principal (MB) avec un affichage (DP), et un processeur (620), le procédé comprenant les étapes consistant à :
recevoir une demande d'estimation de bio-informations ;
commander l'affichage pour afficher un premier objet graphique (811) représentant une apparence du corps principal comportant une partie capteur (SB) et un deuxième objet graphique (812) représentant une apparence d'un doigt pour guider le doigt afin qu'il soit en contact normal avec la partie capteur (SB) ;
acquérir (1310), par la partie capteur (610), un état de contact d'un doigt avant la mesure d'un bio-signal, dans lequel la partie capteur comprend un capteur optique (611) et un capteur de force (612) configuré pour mesurer la force de contact sur la base du doigt contactant la surface de contact de doigt ;
déterminer (1320) si l'état de contact du doigt est normal sur la base d'au moins l'une parmi une analyse déterminant si une force de contact initiale se situe dans une plage seuil prédéfinie, et une analyse déterminant si une force de contact est mesurée pendant une période de temps seuil ;
en réponse à un état de contact qui est normal, commander un affichage, par le processeur (620), pour afficher le premier objet graphique (821), le deuxième objet graphique (822) et un troisième objet graphique (823) pour inciter l'utilisateur à appliquer une pression sur la partie capteur (SB) avec le doigt pour mesurer le biosignal ;
acquérir (1350), par la partie capteur (610), une force de contact du doigt lors de la mesure du bio-signal ;
commander (1360) l'affichage, par le processeur (620), pour afficher un septième objet graphique (1111, 1112) lié à une force de contact de référence à laquelle le doigt appuie sur la partie capteur (SB) et un huitième objet graphique (1121, 1122) représentant une force de contact réelle mesurée par le capteur de force pour inciter l'utilisateur à appuyer sur la partie capteur (SB) avec une force normale prédéfinie ;
acquérir (1370) du bio-signal par la partie capteur (SB) ; et
estimer des bio-informations (1380) sur la base du bio-signal et de la force de contact.

2. Procédé selon la revendication 1 comprenant en outre :
en réponse à l'état anormal du contact, la commande de l'affichage pour afficher au moins l'un d'un cinquième objet graphique (910) indiquant que l'état de contact est anormal, d'un sixième objet graphique (921, 922, 923) affichant visuellement une raison pour laquelle l'état de contact est anormal, et d'un texte (931, 932, 933) décrivant une raison pour laquelle l'état de contact est anormal.

3. Dispositif électronique (600 ; 700) comprenant un corps principal (MB) avec un affichage (DP), une partie capteur (610) disposée sur un côté du corps principal et un processeur (620), dans lequel le dispositif électronique (600 ; 700) est configuré pour effectuer le procédé selon l'une des revendications 1 à 2.

4. Support de stockage non transitoire lisible par ordinateur stockant des instructions qui, lorsqu'elles sont exécutées par un processeur (620) d'un dispositif électronique (600 ; 700) comprenant un corps (MB) avec un affichage (DP), une partie capteur (610) disposée sur un côté du corps principal, et le processeur (620), amènent le processeur (620) à :
recevoir une demande d'estimation de bio-informations ;
commander l'affichage pour afficher un premier objet graphique (811) représentant une apparence du corps principal comportant une partie capteur (SB) et un deuxième objet graphique (812) représentant une apparence d'un doigt pour guider le doigt afin qu'il soit en contact normal avec la partie capteur (SB) ;
acquérir (1310), par la partie capteurs (610), un état de contact d'un doigt avant la mesure d'un bio-signal ;
déterminer si l'état de contact du doigt est normal sur la base d'au moins l'une parmi une analyse déterminant si une force de contact initiale se situe dans une plage de seuil prédéfinie, et une analyse déterminant si une force de contact est mesurée pendant une période de temps seuil ;
en réponse à l'état de contact qui est normal, commander un affichage, par le processeur (620), pour afficher le premier objet graphique (821), le deuxième objet graphique (822), et un troisième objet graphique (823) pour inciter l'utilisateur à appliquer une pression sur la partie capteur (SB) avec le doigt pour mesurer le biosignal ;
acquérir, par la partie capteurs (610), une force de contact du doigt lors de la mesure du bio-signal ;
commander l'affichage, par le processeur (620), pour afficher un septième objet graphique (1111, 1112) lié à une force de contact de référence à laquelle le doigt appuie sur la partie capteur (SB) et un huitième objet graphique (1121, 1122) représentant une force de contact réelle mesurée par le capteur de force pour inciter l'utilisateur à appuyer sur la partie capteur (SB) avec une force normale prédéfinie ;
acquérir le bio-signal par la partie capteur (SB) ; et
estimer des bio-informations sur la base du bio-signal et de la force de contact,
dans lequel la partie capteur (610) comprend un capteur optique (611) comprenant une source lumineuse configurée pour émettre de la lumière vers le doigt en contact avec une surface de contact de doigt et un photodétecteur configuré pour détecter de la lumière diffusée ou réfléchie par le doigt, et un capteur de force (612) configuré pour mesurer la force de contact sur la base du doigt contactant la surface de contact de doigt.

5. Support de stockage lisible par ordinateur selon la revendication 4,
dans lequel le processeur est en outre configuré pour commander l'affichage afin d'afficher le deuxième objet graphique (812) ou un texte (813) pour qu'il apparaisse et disparaisse de manière répétée une ou plusieurs fois à des intervalles de temps spécifiques après une période de temps prédéterminée pendant que le premier objet graphique (811) est affiché.

6. Support de stockage lisible par ordinateur selon la revendication 5, dans lequel le troisième objet graphique (823) représente une apparence du doigt qui se déplace de manière répétée d'une position prédéterminée espacée de la partie capteur (SB) du corps principal à une position de la partie capteur (SB), ou dans lequel le troisième objet graphique (823) comporte une flèche dirigée vers la partie capteur (SB).

7. Support de stockage lisible par ordinateur selon la revendication 6, dans lequel le processeur est en outre configuré pour commander l'affichage afin d'afficher un quatrième objet graphique (825) indiquant que l'état de contact est normal sur la base que l'état de contact est normal, ou
dans lequel le processeur est en outre configuré pour afficher au moins l'un d'un cinquième objet graphique (910) indiquant que l'état de contact est anormal, d'un sixième objet graphique (921, 922, 923) affichant visuellement une raison pour laquelle l'état de contact est anormal, et d'un texte (931, 932, 933) décrivant une raison pour laquelle l'état de contact est anormal sur la base que l'état de contact est anormal.
